# FASCICULE DE BREVET EUROPEEN

(11) **EP 1 678 191 B1**
(45) Date de publication et mention de la délivrance du brevet: **15.08.2007**
(21) Numéro de dépôt: 04805347.4
(22) Date de dépôt: 29.10.2004
(51) Int. Cl.: C07H 3/06, C07H 15/04, C08B 37/00, A61K 31/70, A61K 31/715, A61K 31/48

(54) **PROCEDE DE SYNTHESE DIRECTE D'OLIGORHAMNOSIDES, COMPOSITION COMPRENANT DES OLIGORHAMNOSIDES ET UTILISATION EN TANT QUE MEDICAMENT**
VERFAHREN ZUR DIREKTEN SYNTHESE VON OLIGORHAMNOSIDEN, OLIGORHAMNOSIDHALTIGE ZUSAMMENSETZUNG UND VERWENDUNG DAVON ALS MEDIKAMENT
METHOD FOR THE DIRECT SYNTHESIS OF OLIGORHAMNOSIDES, COMPOSITION COMPRISING OLIGORHAMNOSIDES AND USE THEREOF AS A MEDICAMENT

(30) Priorité: 31.10.2003 FR 0312796
(43) Date de publication de la demande: 12.07.2006
(73) Titulaire: Pierre Fabre Dermo-Cosmétique, 92100 Boulogne-Billancourt (FR); CENTRE NATIONAL DE LA RECHERCHE SCIENTIFIQUE, 75016 Paris Cédex (FR)
(72) Inventeur: HOULMONT, Jean-Philippe, F-31520 Ramonville St Agne (FR); RICO-LATTES, Isabelle, F-31650 Auzielle (FR); PEREZ, Emile, F-31770 Colomiers (FR); BORDAT, Pascal, F-31320 Mervilla (FR)
(74) Mandataire: Ahner, Francis
(86) Numéro de dépôt international: PCT/FR2004/002793
(87) Numéro de publication internationale: WO 2005/042553

(56) Documents cités:
- US-A- 5 635 612
- BEDINI E ET AL: "Oligomerization of a rhamnanic trisaccharide repeating unit of O-chain polysaccharides from phytopathogenic bacteria" TETRAHEDRON LETTERS, ELSEVIER SCIENCE PUBLISHERS, AMSTERDAM, NL, vol. 43, no. 49, 2 décembre 2002 (2002-12-02), pages 8879-8882, XP004391857 ISSN: 0040-4039
- ZHU Y., KONG F. , SYNLETT, no. 12, 2000, pages 1783-1787, XP001205161

## Description

La présente invention concerne un procédé de synthèse directe d'oligorhamnosides. Le procédé de synthèse consiste en une réaction « one-pot » dans l'acétonitrile, sans aucune réaction de protection ou de déprotection du rhamnose. Le mélange d'oligorhamnosides obtenu présente une activité anti-inflammatoire.

La réaction inflammatoire est une réponse du système immunitaire face à une agression causée aux cellules ou aux tissus vascularisés d'un organisme par un pathogène tel que les virus, les bactéries ou bien face à une agression chimique ou physique. Souvent douloureuse, l'inflammation est généralement une réponse de guérison. Cependant dans certains cas, (arthrite rhumatoïde, la maladie de Crohn, les maladies autoimmunes...) elle peut avoir des conséquences plus graves que le stimulus d'origine.

Les réactions d'hypersensibilité de contact correspondent à des réactions d'immunité spécifique dirigées contre des antigènes localisés sur des cellules ou dans les tissus, à l'origine de lésions cellulaires ou de réactions inflammatoires. Ces réactions d'hypersensibilité peuvent se développer dans le cadre des mécanismes de défense vis-à-vis d'un microorganisme pathogène ou dans le cas de réactions allergiques. Elles font intervenir différents types de cellules et en particulier les cellules de la peau, certains leucocytes, sans oublier les cellules endothéliales dont le rôle est prépondérant dans les réactions inflammatoires.

Les interactions intercellulaires qui interviennent alors impliquent généralement des phénomènes de reconnaissances spécifiques entre ligands et récepteurs. Au cours de ces vingt dernières années, de nombreux récepteurs de surface cellulaires ont été identifiés, tels que des protéines capables d'assurer une reconnaissance spécifique avec certains sucres comme le fucose et le rhamnose.

Les lectines sont des protéines implantées dans les membranes des cellules eucaryotes, et jouent un rôle très important dans les phénomènes d'adhésion et de reconnaissance entre cellules notamment lors des processus inflammatoires. Les lectines membranaires sont impliquées en particulier dans l'endocytose, le trafic intracellulaire des glycoconjugués et la perméabilité endothéliale. De plus, ces protéines, souvent transmembranaires, contribuent à la reconnaissance spécifique de l'antigène (domaine extracellulaire) et à l'activation des cellules (domaine intracellulaire). Les lectines peuvent reconnaître spécifiquement certains sucres, notamment le rhamnose.

L'étude, ainsi que l'utilisation thérapeutique d'oligorhamnosides, impliquent de plus en plus de disposer de ces produits en grande quantité. Malheureusement ceux-ci sont difficiles à isoler sous une forme homogène à partir de cellules vivantes, de par le fait qu'ils existent sous la forme de mélanges microhétérogènes. La purification de tels composés, lorsque cela est possible, est difficile et aboutit généralement à de très faibles rendements. Ces contraintes montrent l'intérêt de disposer d'une méthode de synthèse simple et efficace d'oligorhamnosides.

De nombreux chercheurs se sont essayés au cours du siècle dernier à proposer des méthodes originales dans la synthèse d'oligosaccharides. Les premières ont été initiées avec Fischer, Köenigs et Knorr, Lemieux (Lemieux, R.U., Morgan, A.R., The preparation and configurations of tri-O-acetyl-alpha-D-glucopyranose 1,2-(orthoesters). Canadian journal of chemistry, 1965. 43: p. 2199-2204) qui introduirent les notions d'activation et de protection des unités saccharidiques. Ensuite, Paulsen (Paulsen, H., Kutschker, W., Lockhoff, O., Building units for Oligosaccharides, XXXIII: synthesis of beta-glycosidically linked disaccharides of L-rhamnose. Chemical Berstein, 1981. 114: p. 3233-3241), et plus récemment Schmidt et Seeberger (Seeberger, P.H.H.W.C., Solid-phase oligosaccharides synthesis and combinatorial carbohydrate librairies. Chemical reviews, 2000. 100(n°12): p. 4349-4393; Seeberger, P.H., Plante, O.J., Synthesis of oligosaccharides, reagents and methods related thereto. 2001, MIT (Cambridge MA): United States. p. 50) proposèrent des méthodes plus complexes sur support solide afin d'améliorer les rendements et la spécificité des réactions. On peut également citer les méthodes décrites dans le brevet US 5,635,612 ou celle décrite dans Bedini et al, Tetrahedron Letters, Vol 43, n°49, 2.12.02, pages 8779-8882, ou encore celle décrite par Zhu Y. et al, Synlett, n°12, 2000, pages 1783-1787. Dans toutes ces méthodes, les monomères sont protégés, avec un groupement protecteur approprié, avant condensation. La synthèse d'oligosaccharides avait comme objectif d'exploiter leur biocompatibilité afin de trouver de nouvelles substances thérapeutiques. Cependant, ces méthodes reposent sur des stratégies qui nécessitent toutes des étapes de protection et de déprotection des sucres. Ces méthodes de synthèse restent donc lourdes et s'accompagnent malheureusement de rendements très faibles diminuant les possibilités d'industrialisation. Il est donc primordial de trouver un procédé original adapté qui permettrait la synthèse de ces composés avec un minimum d'étapes réactionnelles.

D'une manière surprenante, les inventeurs ont découvert qu'il était possible de synthétiser directement dans l'acétonitrile des oligorhamnosides (synthèse « one-pot ») sans aucune réaction de protection ni de déprotection du rhamnose.

Cette synthèse en une seule étape réactionnelle utilise les propriétés particulières de solubilité du rhamnose dans l'acétonitrile : le rhamnose est très peu soluble dans l'acétonitrile froid et peu soluble à chaud.

Au sens de la présente invention, on entend par « oligorhamnoside » tout oligomère constitué de motifs rhamnoses, de configuration lévogyre ou dextrogyre, avantageusement lévogyre, liés entre eux par des liaisons glycosidiques de configuration *α* ou *β.* Ledit oligomère peut être linéaire ou sous forme ramifiée.

La présente invention concerne un procédé de préparation d'oligorhamnosides comprenant les étapes successives suivantes :
a) auto-condensation du rhamnose en une seule étape réactionnelle dans l'acétonitrile en présence d'un catalyseur acide et précipitation des oligorhamnosides ainsi formés ; puis
b) récupération du précipité obtenu suite à l'étape a), comprenant les oligorhamnosides, par filtration.

L'originalité du procédé tient au fait que la réaction d'auto-condensation du rhamnose dans l'acétonitrile se fasse directement sans étape préliminaire de protection des fonctions hydroxyles du rhamnose et, de la même manière, sans étape successive de déprotection des fonctions hydroxyles du rhamnose.

Lors de ladite étape a), on travaille avantageusement en milieu homogène pour limiter l'excès de rhamnose dans le brut final, tout en travaillant à saturation pour assurer la précipitation rapide des oligorhamnosides. Ainsi, la solution d'acétonitrile est avantageusement saturée en rhamnose.

Le mélange rhamnose, acétonitrile et catalyseur acide est avantageusement mis à réagir sous chauffage et éventuellement sous agitation, à une température comprise entre 20 et 120°C, encore plus avantageusement entre 35 et 75°C. La température d'autocondensation ne doit pas dépasser 120°C afin d'éviter une dégradation des sucres. D'une manière avantageuse, la température de chauffage est d'environ 65°C à pression atmosphérique. Le mélange rhamnose, acétonitrile et catalyseur acide est avantageusement mélangé de 5 minutes à 24 heures, encore plus avantageusement 3 heures.

Dans la chimie des sucres, il est très classique d'utiliser un catalyseur acide pour favoriser la formation de liaisons glycosidiques. Dans le cadre de la présente invention, le catalyseur acide est avantageusement choisi dans le groupe constitué par l'acide chlorhydrique, l'acide sulfurique, l'acide phosphorique, l'acide ortho-, méta-, et para-toluène sulfonique, l'acide benzène-sulfonique, les acides benzène-sulfoniques substitués, l'acide méthane-sulfonique, les acides de Lewis, notamment le chlorure de zinc ou le chlorure ferrique, des argiles acides, notamment la montmorillonite K-10, des résines acides synthétiques, les zéolithes ou leurs combinaisons.

Les acides benzène-sulfoniques substitués sont avantageusement les acides ortho-, méta-, para-bromobenzènesulfonique.

Les acides de Lewis sont par exemple le chlorure de zinc, le chlorure ferrique ou tout autre halogénure de métal, métalloïde ou lanthanide.

Comme exemple de résines acides synthétiques, on peut notamment citer les résines acides synthétiques de type « Amberlyst® », « Amberlite® » ou « Dowex® ».

La quantité de catalyseur doit être contrôlée car la réaction est très rapide. Afin de contrôler davantage la réaction, il est nécessaire de ralentir cette réaction. La quantité de catalyseur acide ajoutée est avantageusement fixe. La quantité de catalyseur ajoutée correspond avantageusement à environ 0,1 mole de catalyseur pour 1 mole de rhamnose.

L'eau formée pendant la réaction d'auto-condensation de l'étape a) est avantageusement éliminée, physiquement ou chimiquement. Comme exemple de technique d'élimination physique de l'eau formée pendant la synthèse, on peut notamment citer la distillation ou l'utilisation d'un adsorbant. Comme exemple de technique d'élimination chimique de l'eau formée pendant la synthèse, on peut notamment citer l'utilisation d'un agent de dessiccation.

Selon une variante avantageuse de l'invention, l'eau formée pendant la réaction d'auto-condensation de l'étape a) est éliminée au moyen d'un agent de dessiccation choisi dans le groupe constitué par les carbonates, les sulfates, le chlorure de calcium, le pentaoxyde de phosphore, des tamis moléculaires ou des combinaisons de ces divers agents de dessiccation. L'agent de dessiccation peut être introduit directement dans le milieu réactionnel ou être présent au niveau des vapeurs de solvant à l'intérieur d'une cartouche d'extraction solide/liquide de type « soxhlet ».

Selon une variante de l'invention, la réaction d'auto-condensation de l'étape a) est réalisée à pression atmosphérique et sous atmosphère d'un gaz inerte, tel que l'argon ou l'azote.

Selon une autre variante de l'invention, la réaction d'auto-condensation de l'étape a) est réalisée à pression réduite (de préférence environ 260 mbar), avantageusement en autoclave.

Les réactifs peuvent être introduits de manière fractionnée ou continue de façon à ce que la réaction puisse toujours être réalisée dans les rapports molaires les plus favorables.

Préalablement à l'étape b), le mélange réactionnel est avantageusement refroidi jusqu'à une température comprise entre la température de réaction de condensation et 0°C, encore plus avantageusement jusqu'à la température ambiante, c'est-à-dire environ jusqu'à 20°C.

Cette étape additionnelle de refroidissement favorise la précipitation des oligorhamnosides formés lors de l'étape a).

Le précipité récupéré lors de l'étape b) est appelé précipité P1. Il est avantageusement récupéré par filtration telle qu'une filtration sur Büchner.

Ledit précipité P1 est avantageusement lavé à l'acétonitrile. Le filtrat obtenu suite à l'étape b) est évaporé sous pression réduite afin de récupérer un second précipité (P2) contenant le rhamnose n'ayant pas réagi mais aussi des dérivés rhamnosylés du précipité P1 passés en solution.

La précipitation d'oligorhamnosides au cours de la réaction libère des molécules de solvant (acétonitrile). Ainsi, il se produit de manière réversible une solubilisation des plus faibles masses, et c'est pourquoi le précipité P2 obtenu par évaporation de la phase acétonitrile contient une partie riche de rhamnose mais également une fraction des oligorhamnosides synthétisés.

Les oligorhamnosides obtenus présentent un degré de polymérisation maximum égal à 12, avantageusement compris entre 2 et 9.

On pense que la modification des conditions opératoires ne contribue pas au changement de la distribution des masses mais uniquement à une modification de la cinétique de formation des oligorhamnosides. En effet, si on considère qu'à partir d'une certaine taille, un oligomère n'est plus soluble dans l'acétonitrile et précipite, alors ce phénomène peut être accompagné de deux autres phénomènes sous jacents et opposés. Le premier est la libération de molécules de solvant qui seront alors disponibles pour solubiliser davantage les oligomères de plus petites tailles, et le second est un phénomène de coprécipitation. Un oligomère de masse suffisamment importante peut provoquer la précipitation de ses homologues plus petits lorsqu'il devient insoluble dans le milieu réactionnel.

Ces trois phénomènes, de précipitation, coprécipitation et resolubilisation, sont probablement à l'origine d'une uniformité qualitative, dans la distribution de masse.

Les motifs rhamnose ont jusqu'à trois de leurs fonctions hydroxyles qui sont impliquées dans la formation de liaisons glycosidiques. En théorie, le motif rhamnose ayant quatre fonctions hydroxyles, il pourrait former quatre liaisons glycosidiques. Cependant, lors de la mise en oeuvre du procédé selon l'invention, on a constaté qu'au maximum trois des fonctions hydroxyles des motifs rhamnose sont impliquées dans la formation de liaisons glycosidiques. On pense que ceci est du à des problèmes d'encombrements stériques.

Le rendement massique en oligorhamnosides du procédé selon l'invention est compris entre 30 et 60%, par rapport à la quantité massique de rhamnose introduite.

La présente invention concerne également une composition comprenant un mélange d'oligorhamnosides susceptible d'être obtenu par le procédé selon l'invention, lesdits oligorhamnosides contenant de 2 à 12 motifs rhamnose, avantageusement de 2 à 9 motifs rhamnose.

La distribution des oligorhamnosides en fonction de leur degré de polymérisation suit approximativement une loi de distribution du type loi de Poisson.

Les motifs rhamnoses ont jusqu'à trois de leurs fonctions hydroxyles qui sont impliquées dans la formation de liaisons glycosidiques. Les liaisons glycosidiques peuvent être des liaisons en α ou des liaisons en β.

La présente invention concerne aussi un médicament comprenant une composition selon l'invention, c'est-à-dire un mélange d'oligorhamnosides tel que défini précédemment.

Le médicament selon l'invention est avantageusement destiné à réguler les mécanismes inflammatoires.

En particulier, le médicament est destiné à la prévention ou au traitement des réactions ou pathologies allergiques, inflammatoires, immunitaires de la peau et/ou des muqueuses. Le médicament selon l'invention est également destiné à inhiber la réponse immune liée au stress inflammatoire.

Le médicament selon l'invention est notamment destiné à inhiber l'activation des leucocytes, tels que les polynucléaires humains et notamment les neutrophiles et les mastocytes humains empêchant la libération des médiateurs préformés de la réaction immunitaire. Il permet également l'inhibition de l'adhésion des lymphocytes circulants et des cellules endothéliales empêchant ainsi la transmigration de ces leucocytes sur le lieu de l'inflammation. Il permet aussi l'inhibition de la sécrétion des cytokines kératinocytaires, activatrices des lymphocytes T et des cellules de Langerhans, telles que IL-1, TNF-α, ou des molécules d'adhésion, telles que ICAM-1, VCAM, qui contibuent au recrutement et au passage trans-endothélial des leucocytes. Le médicament selon l'invention est également inhibiteur du phénomène d'hyperplasie kératinocytaire.

Le médicament selon l'invention est aussi inhibiteur du processus d'apprêtement de l'antigène par les cellules dendritiques de la peau, de la maturation des cellules présentatrices d'antigène, à savoir les cellules dendritiques dermiques et les cellules de Langerhans, du phénomène de reconnaissance entre les lymphocytes et les cellules présentatrices d'antigène.

Ainsi, le médicament selon l'invention est destiné à la prévention ou au traitement des maladies choisies dans le groupe constitué de l'eczéma atopique et/ou de contact, des dermatoses inflammatoires, des dermatites irritatives, de l'acné, des maladies auto-immunes telles que le psoriasis, de la photo-immuno-suppression, du vitiligo, du pityriasis, des sclérodermies, de l'arthrite rhumatoïde, de la maladie de Crohn ou du rej et de greffe.

Le médicament selon l'invention est également destiné à la prévention et au traitement des dérives inflammatoires chroniques liées au vieillissement et ses conséquences. Le médicament est notamment destiné à la prévention ou au traitement des maladies choisies dans le groupe constitué par les sensibilisations anaphylactiques, les anomalies pigmentaires de la peau, l'hypervascularisation dermique, les fissurations inflammatoires.

Selon une variante de l'invention, le médicament est destiné à diminuer le caractère allergisant et/ou irritant d'une composition ou d'un parfum.

Le médicament selon l'invention comprend avantageusement 0,001 à 50% en poids d'oligorhamnosides.

Le médicament selon la présente invention peut être formulé pour être administré par toute voie. Il est avantageusement formulé pour être administré par voie topique, orale, sous-cutanée, injectable, rectale et génitale.

Lorsque le médicament est formulé pour être administré par voie orale, ledit médicament peut se présenter sous la forme de solution aqueuse, émulsion, comprimés, gélules, capsules, poudres, granules, solutions ou encore de suspensions orales.

Lorsque le médicament est formulé pour être administré par voie sous-cutanée, ledit médicament ou ladite composition peut se présenter sous la forme d'ampoules injectables stériles.

Lorsque le médicament est formulé pour être administré par voie rectale, ledit médicament peut se présenter sous la forme de suppositoires.

Lorsque le médicament est formulé pour être administré par voie génitale, ledit médicament peut se présenter sous la forme d'ovules.

Le médicament selon l'invention est de préférence à application topique. Le médicament peut alors être formulé de manière à se présenter par exemple sous forme de solution aqueuse, de crème blanche ou colorée, de pommade, de lait, de lotion, de gel, d'onguent, de sérum, de pâte, de mousse, d'aérosol ou de stick.

La quantité du médicament selon l'invention à administrer dépend de la gravité et de l'ancienneté de l'affection traitée. Naturellement, le médecin adaptera aussi la posologie en fonction du malade.

La présente invention concerne également une méthode de traitement cosmétique des peaux et/ou des muqueuses sensibles, irritées, intolérantes, à tendance allergique, âgées, soumises à un signal danger, présentant un trouble de la barrière cutanée, présentant des rougeurs cutanées ou présentant un déséquilibre immunologique non pathologique lié au vieillissement intrinsèque, extrinsèque ou hormonal, caractérisée en ce qu'elle consiste à appliquer sur la peau et/ou les muqueuses une composition comprenant un mélange d'oligorhamnosides tel que défini précédemment.

La présente invention concerne aussi une méthode de traitement cosmétique pour retarder le vieillissement naturel de la peau et/ou pour prévenir le vieillissement accéléré de la peau soumise aux agressions extérieures, notamment pour prévenir le vieillissement photo-induit de la peau, caractérisée en ce qu'elle consiste à appliquer sur la peau une composition comprenant un mélange d'oligorhamnosides tel que défini précédemment.

Dans le cadre d'une utilisation cosmétique, la composition selon l'invention comprend avantageusement 0,001 à 50% en poids d'oligorhamnosides, par rapport au poids total de la composition.

Lorsque la composition cosmétique est formulée pour être administré par voie topique, ladite composition se présente par exemple sous forme de solution aqueuse, de crème blanche ou colorée, de pommade, de lait, de lotion, de gel, d'onguent, de sérum, de pâte, de mousse, d'aérosol, de shampoing ou de stick.

D'autres caractéristiques et avantages de l'invention apparaissent dans la suite de la description avec les exemples représentés ci-après. Dans ces exemples on se référera aux figures suivantes. Ces figures et exemples sont destinés à illustrer la présente invention et ne peuvent en aucun cas être interprétés comme pouvant en limiter la portée.
- **Figure 1** :: Viabilité des cellules endothéliales issues des ganglions lymphatiques périphériques en présence de rhamnose.
- **Figure 2** :: Viabilité des cellules endothéliales issues des ganglions lymphatiques périphériques en présence des oligorhamnosides.

### Exemple 1 : Synthèse directe des oligorhamnosides

Dans un ballon à 2 voies de 100 mL, surmonté d'un réfrigérant droit lui-même équipé d'une garde desséchante (CaCl₂), on introduit sous balayage d'argon, 25 mL d'acétonitrile (séché sur tamis moléculaire 3 Å). Le rhamnose (360 mg) est introduit dans l'acétonitrile à chaud (65 °C), en quatre fractions équivalentes, en attendant la complète dissolution avant d'ajouter la fraction suivante.

Le catalyseur acide p-toluène sulfonique (APTS), 0,3 mL d'une solution à 0,6 M (rapport massique de 0,1 g/g de rhamnose) est ajouté dans le mélange maintenu sous argon. La solution est agitée (agitateur magnétique) à 65°C pendant 40 minutes.

Après réaction, l'agitation est coupée et le mélange refroidi à température ambiante pendant 30 minutes.

Le précipité (P1) formé est récupéré par filtration sous vide sur un verre fritté de porosité 4, puis est lavé à 1' acétonitrile et à l'éther anhydre, puis repris dans un minimum d'eau et lyophilisé. On obtient ainsi 126 mg d'une poudre blanche correspondant aux oligorhamnosides, soit un rendement de 35 %.

Le filtrat récupéré est évaporé à l'évaporateur rotatif sous pression réduite (15 mmHg), on obtient ainsi 234 mg d'un solide (P2) correspondant à un mélange rhamnose+oligorhamnosides de faibles masses moléculaires. La distribution de taille ainsi que les masses des oligosaccharides synthétisés sont respectivement mesurées par HPLC et par spectrométrie de masse.

Les oligorhanmosides (P1) et (P2) sont analysés par chromatographie liquide haute performance (HPLC) sur une colonne RSO-oligosaccharides, REZEX de marque Phenomex (200x10,0 mm) munie d'une colonne de garde (60x10,0 mm). Eluant 100 % H₂O à un débit de 0,3 mL/min et à une température de colonne de 80°C. Les échantillons injectés à 10 µL d'une concentration de 50 mg/mL dans l'eau, sont détectés par un réfractomètre différentiel. Dans ces conditions, l'analyse dure 50 minutes et permet de séparer le rhamnose (47 minutes de temps de rétention) des dérivés rhamnosylés (temps de rétention de 15 à 44 minutes).

Les masses des oligorhamnosides (P1) et (P2) sont déterminées par spectrométrie de masse, électrospray pour les degrés de polymérisation de 1 à 5 et LSI-MS pour les degrés de polymérisation de 5 à 12. Ces analyses de masses font apparaître un degré maximum de polymérisation égale à 12. Tous les types de liaisons glycosidiques sont trouvés, excepté le motif rhamnose où toutes les fonctions hydroxyles ont réagi.

### Exemple 2 : Synthèse directe des oligorhamnosides

On procède selon le protocole de l'exemple 1, mais dans ce cas la solution est agitée à 40 °C pour une durée de 40 minutes comme selon l'exemple 1.

Après réaction et traitements suivant les mêmes protocoles que ceux décrits à l'exemple 1, on obtient 72 mg de précipité (P1) soit un rendement de 20 %.

La masse de solide (P2) récupérée étant dans cet exemple de 288 mg.

Les analyses HPLC et spectrométrie de masse conduisent aux mêmes résultats que ceux obtenus selon le protocole de l'exemple 1.

### Exemple 3 : Synthèse directe des oligorhamnosides

On procède selon les protocoles des exemples 1 et 2, mais dans ce cas la solution est agitée à 65 °C, mais pour une durée de 6 heures. Après réaction et traitements suivant les mêmes protocoles que ceux décrits à l'exemple 1 et 2, on obtient 144 mg d'un précipité brun-jaune (P1) soit un rendement de 40 %.

La masse de solide (P2) récupérée étant dans cet exemple de 216 mg.

Les analyses HPLC et spectrométrie de masse conduisent mêmes résultats que ceux obtenus selon le protocole de l'exemple 1 et 2.

### Exemple 4 : Synthèse directe des oligorhamnosides

On procède selon les protocoles des exemples 1, 2, 3, mais dans ce cas la solution est agitée à 40 °C pour une durée de 6 heures. Après réaction et traitements suivant les mêmes protocoles que ceux décrits dans les exemples 1 à 3, on obtient 126 mg d'un précipité (P1) soit un rendement de 35 %.

La masse de solide (P2) récupérée étant dans cet exemple de 234 mg.

Les analyses HPLC et spectrométrie de masse conduisent aux mêmes résultats que ceux obtenus selon les protocoles 1 à 3.

### Exemple 5 : Synthèse directe des oligorhamnosides

On procède selon le protocole de l'exemple 1, mais dans ce cas la quantité totale de rhamnose introduite dans l'acétonitrile à 65 °C est de 200 mg.

Après réaction et traitements suivant les mêmes protocoles que ceux décrits dans les exemples 1 à 4, on obtient 30 mg de précipité (P1) soit un rendement de 15%.

La masse de solide (P2) récupéré étant dans cet exemple de 170 mg.

Les analyses HPLC et spectrométrie de masse conduisent aux mêmes résultats que ceux obtenus selon les protocoles 1 à 4.

### Exemple 6 : Synthèse directe des oligorhamnosides

On procède selon le protocole de l'exemple 1, mais dans ce cas la quantité de catalyseur APTS est plus importante, soit un rapport massique de 0,2 g/g de rhamnose.

Après réaction et traitements suivant les mêmes protocoles que ceux décrits dans les exemples 1 à 5, on obtient 126 mg de précipité (P1) soit un rendement de 35 %.

La masse de solide (P2) récupéré étant dans cet exemple de 234 mg.

Les analyses HPLC et spectrométrie de masse conduisent aux mêmes résultats que ceux obtenus selon les protocoles 1 à 5.

### Exemple 7 : Synthèse directe des oligorhamnosides

On procède selon le protocole de l'exemple 1, mais sur un volume d'acétonitrile et une masse de rhamnose plus importante et une durée plus longue.

Dans un ballon à deux voies de 100 mL, surmonté d'un réfrigérant droit, lui- même équipé d'une garde desséchante (CaCI₂), on introduit sous balayage d'argon, 80 mL d'acétonitrile sec. Le rhamnose (550 mg) est introduit dans l'acétonitrile chaud (65 °C) par petites fractions. Dans ce cas la solution est agitée à 65°C pour une durée de 4 heures.

Après réaction et traitements suivant les mêmes protocoles que ceux décrits dans les exemples 1 à 6, on obtient 137,5 mg de précipité (P1) soit un rendement de 25 %.

La masse de solide (P2) récupéré étant dans cet exemple de 412,5 mg.

Les analyses HPLC et spectrométrie de masse conduisent aux mêmes résultats que ceux obtenus selon les protocoles 1 à 6.

### Exemple 8 : Synthèse directe des oligorhamnosides

On procède selon le protocole de l'exemple 1, mais en remplaçant l'acide p- toluène sulfonique (APTS) par une résine acide comme l'Amberlyst®15 dry.

Ce catalyseur (500 mg) est ajouté dans le mélange maintenu sous argon.

Après réaction et traitements suivant les mêmes protocoles que ceux décrits dans les exemples 1 à 7, on obtient 90 mg de précipité (P1) soit un rendement de 25%.

La masse de solide (P2) récupéré étant dans cet exemple de 270 mg.

Les analyses HPLC et spectrométrie de masse conduisent aux mêmes résultats que ceux obtenus selon les protocoles 1 à 7.

### Exemple 9 : Synthèse directe des oligorhamnosides

Un ballon à deux voies de 100 mL est surmonté d'un extracteur solide/liquide de type « soxhlet» muni d'une cartouche d'extraction, lui-même surmonté d'un réfrigérant droit équipé d'une prise de vide. La cartouche d'extraction est remplie de chlorure de calcium sec dans un rapport massique CaCl₂/rhamnose=20 et surmontée d'un coton de verre.

Dans le ballon on introduit sous balayage d'argon, 80 mL d'acétonitrile (séché sur tamis moléculaire 3 Å).

Le rhamnose (550 mg) est introduit dans l'acétonitrile à chaud (65 °C), en quatre fractions équivalentes en attendant la complète dissolution avant d'ajouter la fraction suivante.

Le catalyseur acide *p*-toluène sulfonique (APTS), 1 mL d'une solution dans l'acétonitrile à 0,6 M (rapport massique 0,2 g/g de rhamnose) est ajouté dans le mélange maintenu sous argon.

Le montage est ensuite connecté à une source de vide (trompe à eau) munie d'un régulateur de pression afin d'atteindre 260 mbar. Une fois la pression atteinte, la solution est agitée (agitateur magnétique) à 65 °C, température correspondant au reflux de l'acétonitrile à la pression choisie. Le vide et la température sont maintenus pendant 4 heures, ce qui correspond à plusieurs cycles de remplissage et de siphonnage de l'extracteur.

Après réaction, le vide et l'agitation sont coupés, et le mélange refroidi à température ambiante pendant 30 minutes.

Après réaction et traitements suivant les mêmes protocoles que ceux décrits aux exemples 1 à 8, on obtient 275 mg de précipité (P1) soit un rendement de 50%.

La masse de solide (P2) récupérée étant dans cet exemple de 275 mg.

Les analyses HPLC et spectrométrie de masse conduisent aux mêmes résultats que ceux obtenus selon les protocoles 1 à 8.

### Exemple 10: analyse pharmacologique des oligorhamnosides

Les différentes cellules de l'immunité intervenant dans ces processus de l'inflammation ont été étudiées. Ce sont les cellules dendritiques de la peau, les cellules endothéliales, certains leucocytes et les kératinocytes.

### 1) Principes des techniques de mesure de la viabilité cellulaire

### ❖ technique de réduction du MTT, [3-(4,5-dimethyltiazol-2-yl)-2,5-diphenyl tetrazolium bromide] (commercialisé par Sigma)

Cette technique correspond à un test colorimétrique permettant la quantification des cellules vivantes, métaboliquement actives de manière non radioactive. Le MTT est une molécule cationique qui se fixe aux membranes des mitochondries de façon potentiel-dépendant. Au niveau des mitochondries le MTT sera réduit en bleu de formazan par la déshydrogénase mitochondriale. Les cellules vivantes se colorent donc en bleu, à l'inverse des cellules mortes qui restent transparentes. La mesure de la viabilité est ensuite réalisée par mesure de la densité optique à l'aide d'un lecteur automatique.

Toutefois cette méthode d'analyse semble être mieux adaptée aux cellules adhérentes (type kératinocytes) que pour les cellules non adhérentes (monocytes et cellules dendritiques). Une autre étude a donc été envisagée pour conclure sur la cytotoxité des oligorhamnosides vis-à-vis des cellules différenciées analysées : la cytométrie de flux en présence d'iodure de propidium.

### ❖ technique de réduction du sel de tétrazolium XTT.

Il s'agit d'une technique permettant une quantification de la prolifération cellulaire et du nombre de cellules vivantes, (métaboliquement actives) sans incorporation d'isotopes radioactifs. Le XTT, de couleur jaune, est une molécule cationique qui se fixe aux membranes des mitochondries de façon potentiel-dépendant, comme le fait le MTT.

Au niveau des mitochondries, le XTT sera réduit en formazan (orange) par la tétrazolium réductase mitochondriale. Cette méthode, plus onéreuse que la méthode du MTT, ne nécessite pas dans son protocole la lyse des cellules par le SDS pour libérer le colorant. En effet le produit de réduction est soluble au sein de la cellule. La méthode est ainsi plus rapide. Les cellules vivantes, en absence et en présence d'un traitement avec un produit se colorent, à l'inverse des cellules mortes qui restent incolores. Le taux de formazan produit est détecté au spectrophotomètre pour une longueur d'onde de 450 nm et est directement proportionnel au nombre de cellules métaboliquement actives.

### 2) test de toxicité

❖ Des kératinocytes sont isolés et mis en culture à partir de biopsie de peau humaine. Les mesures de densité optique (absorbance) des 4 puits traités avec la même concentration de produits sont moyennées. Cette moyenne est comparée à la moyenne des mesures obtenues pour les 4 puits témoin (test t de Student -comparaison des moyennes- différence significative à 95 % si p<0,05 et à 99 % si p<0,01).
Les viabilités des cellules traitées sont exprimées en pourcentage par rapport au témoin (cellules non traitées) de 100 % (DO traité/ DO témoin x 100).
Le rhamnose ne présente pas de cytotoxicité (cf. tableau 1), même pour les concentrations les plus élevées.

**Tableau 1 : Viabilité des kératinocytes en présence de différentes concentrations en rhamnose.**

| | Témoin | Rhamnose | Rhamnose | Rhamnose | Rhamnose |
|---|---|---|---|---|---|
| | | 1 mg/mL | 0,1 mg/mL | 0,01 mg/mL | 0,001 mg/mL |
| % viabilité | 100 | 104 | 98 | 100 | 95 |
| p (Student) | | 0,504 | 0,679 | 0,991 | 0,407 |

Les oligorhamnosides présentent une toxicité aux fortes concentrations, supérieures à 5 mg/mL (cf. tableau 2).

**Tableau 2 : Viabilité des kératinocytes en présence de différentes concentrations en oligorhamnosides.**

| | Témoin | Oligo rhamnosides | Oligo rhamnosides | Oligo rhamnosides | Oligo rhamnosides | Oligo rhamnosides |
|---|---|---|---|---|---|---|
| | | 5mg/mL | 2mg/mL | 1mg/mL | 0,1mg/mL | 0,01mg/mL |
| % Viabilité | 100 | 75 | 92 | 92 | 102 | 117 |
| p (Student) | | <0,01 | 0,072 | 0,104 | 0,554 | <0,01 |

★ Des cellules endothéliales ont été mises en culture, immortalisées et stabilisées dans leur phénotype. Les lignées cellulaires étudiées sont les cellules endothéliales d'appendice, les cellules endothéliales microvasculaire de cerveau, les cellules endothéliales de ganglions lymphatiques mésenthérique, les cellules endothéliales de ganglions lymphatiques périphériques, les cellules endothéliales microvasculaires de la peau.

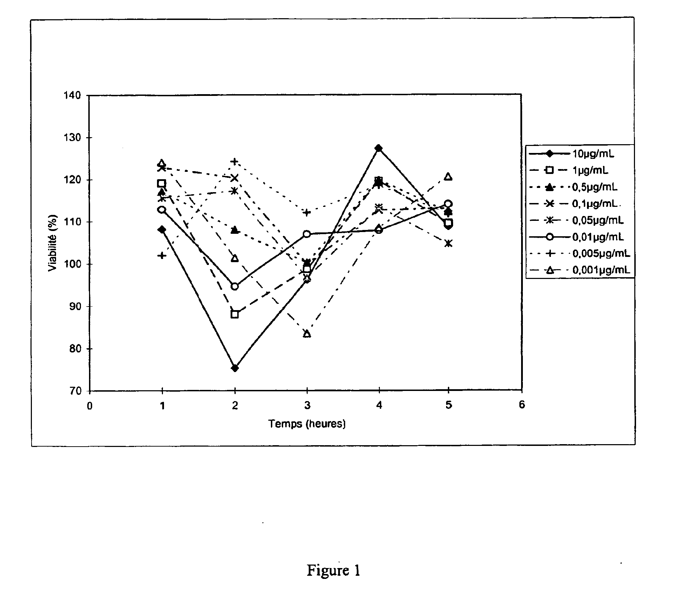

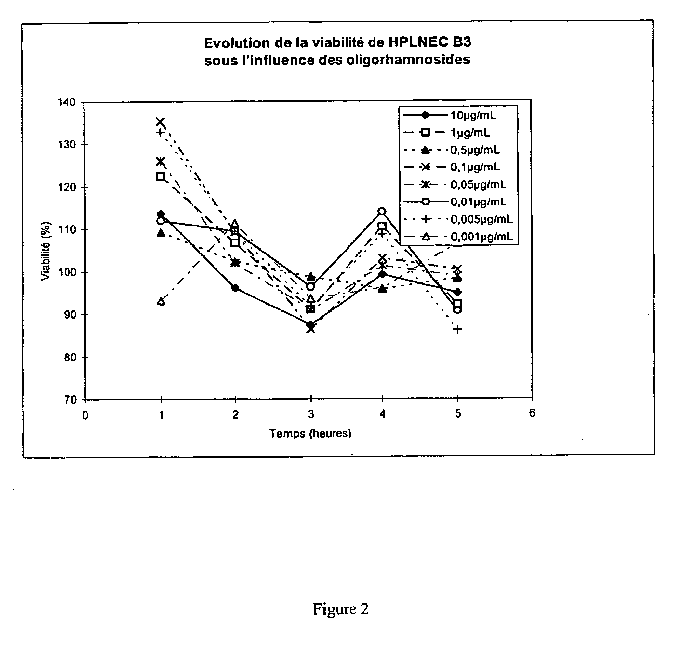

Le test de cytotoxicité est réalisé au moyen d'un test biochimique sur la transformation d'un sel de tétrazolium, le MTT. Les résultats obtenus sont très positifs, aucune toxicité n'est mise en évidence, que ce soit sur le rhamnose ou les oligorhamnosides testés (cf. figures 1 et 2). La viabilité est en effet toujours supérieure à 85 %, et ceci pour toutes les lignées de cellules étudiées.
- **Figure 1** :: Viabilité des cellules endothéliales issues des ganglions lymphatiques périphériques en présence de rhamnose.
- **Figure 2** :: Viabilité des cellules endothéliales issues des ganglions lymphatiques périphériques en présence des oligorhamnosides.

On note en particulier l'apparition d'un pic de stimulation correspondant à 4 heures d'incubation, temps nécessaire pour l'initiation de la synthèse protéique. La présence de ce pic est intéressante car il traduit que les cellules tolèrent les oligorhamnosides (absence de toxicité), et les assimilent. Ces produits semblent enrichir le milieu de culture.

Ces résultats sont similaires pour les autres lignées de cellules endothéliales.

### 3) Influence des oligorhamnosides sur des cellules humaines cultivées en milieu pro-inflammatoire

### ● Sur les cellules dendritiques

On analyse trois groupes de cellules, 1) celles qui n'ont jamais vu ni les oligorhamnosides, ni les signaux d'activation sélectionnés, elles serviront de témoin négatif, 2) celles qui ont été incubées pendant 24 heures avec les signaux d'activation, elles serviront de témoin positif, et enfin 3) celles qui ont été en contact pendant 24 heures avec les oligorhamnosides.

Le milieu de culture utilisé est un milieu classique de type RPMI 1640 (Bioproducts), additionné de SVF 10 % décomplémenté de glutamine (2 mM) et d'antibiotiques (pénicilline et streptomycine, Bioproducts). Le liquide de rinçage utilisé est un tampon PBI (Bioproducts). L'activateur cellulaire utilisé est l'INFγ (SIGMA).

Les résultats sont donnés dans le tableau 3 suivant :

**Tableau 3**

| | Témoin DMSO | | Témoin LPS | | Témoin nul | |
|---|---|---|---|---|---|---|
| Viabilité | 1,27% | | 85,63% | | 92,6% | |
| (Iodure de propidium, PI) | | | | | | |
| CD86 B7.2 | 0,17% | | 49,96% | | 7,4% | |
| (Maturation) | | | | | | |
| | 10mg/mL | 5mg/mL | 1mg/mL | 0,5mg/mL | 0,1mg/mL | 0,05mg/mL |
| Viabilité | 90% | 91% | 91% | 92% | 93% | 93% |
| (Iodure de propidium, PI) | | | | | | |
| CD86 B7.2 | 10% | 6% | 4% | 4% | 5% | 3% |
| (Maturation) | | | | | | |

La viabilité est déterminée à partir de la mortalité traduite directement par la proportion de signal PI. Les résultats présentés dans le tableau 3 traduisent la viabilité cellulaire (100-mortalité), proportion de cellules vivantes dans chaque puit étudié.

L'effet allergisant est traduit par la maturation des cellules dendritiques et par l'expression du récepteur CD86 B7.2. Plus ce récepteur est exprimé, plus la proportion de cellules matures est importante, et plus le dérivé rhamnosylé étudié a des effets allergisants.

Les oligorhamnosides ne provoquent ni mort cellulaire, ni activation ou maturation accélérée des cellules dendritiques. En effet, les cellules dendritiques étudiées continuent à exprimer les récepteurs spécifiques des cellules dendritiques immatures ou ne présentent pas une augmentation de l'expression des autres marqueurs membranaires témoins d'une maturation. En particulier les résultats sont négatifs en ce qui concerne la densité élevée des molécules CD86 caractéristiques des cellules dendritiques matures, les cellules dendritiques n'ont donc pas considéré les oligorhamnosides testés comme des signaux d'activation capables d'accélérer ou de transformer un DC immature en DC activée voire mature.

### ● Dosage de l'IL-8 libérée par les kératinocytes stimulées avec l'activateur IL-1β

Le dosage de l'IL-8 permet d'évaluer les propriétés anti-inflammatoires des oligorhamnosides lorsqu'ils sont ajoutés simultanément à l'activateur utilisé : l'IL-1β. Ce dosage est réalisé sur le surnageant récupéré après le traitement. En effet, les kératinocytes humains normaux (KHN) mis en contact avec l'activateur IL-1β sécrètent de l'IL-8 et sa concentration dans les surnageants cellulaires passe de 3,2 pg/mL/µg protéines pour des KHN non traités à environ 32 pg/mL/µg protéines pour ceux qui sont activés.

Pour affiner le dosage de la cytokine, un dosage supplémentaire est réalisé, celui des protéines cellulaires. Il permettra d'exprimer, pour chaque puits, la quantité d'IL-8 sécrétée par µg de protéines cellulaires (méthode BCA décrite en partie expérimentale).

Les kératinocytes sont traités 24 heures avec oligorhamnosides et avec l'agoniste IL-1β. Après 24 heures, la cytokine IL-8 libérée dans les surnageants cellulaires est quantifiée au moyen d'un test ELISA (Enzyme Linked Immunosorbent Assay).

La gamme standard du kit commercial permet de faire correspondre l'absorbance mesurée à 450nm et la concentration en IL-8 (pg/mL) présente dans chacun des surnageants cellulaires.

Les différentes concentrations testées sont évaluées sur 4 puits cellulaires. On obtient ainsi 4 valeurs distinctes de concentration en IL-8 (pg/mL) pour chaque condition de traitement. Ces quantifications peuvent être rapportées à la quantité de protéines par puits.

Une première expérience consiste à incuber l'IL-1β et les oligorhamnosides en même temps pendant 24 heures. Les résultats ci-après (cf. tableau 4) représentent la moyenne de ces 4 valeurs et l'écart type.

**Tableau 4 : Résultats du traitement des KHN avec l'IL-1β et différentes concentrations en oligorhamnosides pendant 24 heures.**

| Traitement | Moy. IL-8 (pg/mL/µg protéines) | % inhibition |
|---|---|---|
| Témoin négatif | 3,6 ± 0,9 | |
| Témoin positif (IL-1β) | 32,3 ± 8,7 | |

| Stimulation IL-1β, 1 ng/mL | | |
|---|---|---|
| 1 mg/mL | 23,5 ± 6,6 | 27 % |
| 0,1 mg/mL | 31,7 ±5,5 | / |
| 0,01 mg/mL | 31 ±9,3 | / |
| 0,001 mg/mL | 30,1 ± 5,9 | / |

Les résultats montrent que la mise en contact directe des oligorhamnosides avec l'activateur contribue à son inhibition, (27 %, 1 mg/mL) (cf. tableau 5) et que cet effet n'est plus remarqué quand la concentration en oligorhamnosides diminue.

Une seconde expérience est réalisée où les oligorhamnosides sont utilisés en prétraitement pendant 8 heures sur les KHN avant d'ajouter l'IL-1β et les oligorhamnosides en même temps pendant 24 heures.

Comme précédemment, les deux dosages sont réalisés (IL-8 et protéines). Les résultats sont résumés dans le tableau précédent (cf. tableau 5).

**Tableau 5 : Résultats du traitement des KHN avec l'IL-1β et différentes concentrations en oligorhamnosides pendant 24 heures après 8 heures de prétraitement avec les oligorhamnosides.**

| Traitement | Moy. IL-8 (pg/mL/µg protéines) | % inhibition |
|---|---|---|
| Témoin négatif | 5,5 ± 0,9 | |
| Témoin positif (IL-1β) | 141,0 ± 12,7 | |

| Stimulation IL-1β, lng/mL | | |
|---|---|---|
| 1 mg/mL | 101,3 ±16 | 28% |
| 0,1 mg/mL | 117,0 ± 19,8 | 17% |
| 0,001 mg/mL | 116,9 ± 15 | 17% |

Les résultats montrent que la mise en contact en condition de prétraitement des KHN avec les oligorhamnosides ne contribue pas vraiment à une augmentation de l'inhibition, (28 %, 1 mg/mL). Cependant cette inhibition est maintenant observable à plus faible concentration. Ceci nous permet de conclure que le prétraitement avec les oligorhamnosides favorise l'inhibition de la libération d'IL-8 dans le surnageant.

Nous pouvons supposer que les oligorhamnosides occupent les sites réactionnels des kératinocytes conduisant à l'inhibition de la réaction secondaire (blocage des sites de l'IL-1β), cependant nous ne pouvons pas conclure sur le mécanisme d'inhibition du processus inflammatoire.

### ● Dosage de la prostaglandine, 6ketoPGF_{1α}, après stimulation des kératinocytes par le PMA.

La prostaglandine I2 (PGI₂) est un métabolite instable de la voie de dégradation de l'acide arachidonique. Comme la prostaglandine E2, il s'agit d'un médiateur de l'inflammation aux propriétés vasodilatatrices. L'instabilité de la PGI₂ réside dans sa conversion rapide (hydratation non enzymatique) en 6-keto prostaglandine F_{1α} (6-ketoPGF_{1α}). C'est donc cette prostaglandine qui est messurée en tant que marqueur de la synthèse de PGI₂.

**Tableau 6: Résultats du traitement des KHN avec l'IL-1β, le PMA et différentes concentrations en oligorhamnosides après 4 heures de prétraitement avec les oligorhamnosides**

| Traitement | Moy. 6-ketoPGF_{1α} | % inhibition |
|---|---|---|
| | (pg/mL/µg protéines) | |
| Témoin négatif | 24,4 ± 5,5 | |
| Témoin positif (IL-1β 1 ng/mL) | 27,3 ± 2,2 | |
| Témoin positif (PMA 10 ng/mL) | 33,5 ± 2,3 | |

| Stimulation 2 heures IL-1β 1 ng/mL | | |
|---|---|---|
| 1 mg/mL | 23,5 ±1,2 | 14 % |
| 0,1 mg/mL | 21,8 ±0,8 | 20 % |
| 0,001 mg/mL | 25,4 ± 3,6 | / |
| Indométhacine 1 µM | 22,6 ±3,4 | 17 % |

| Stimulation 2 heures PMA 10 ng/mL | | |
|---|---|---|
| 1 mg/mL | 27,5±4,9 | 18 % |
| 0,1 mg/mL | 27,4±4,6 | 18 % |
| 0,001 mg/mL | 29,4±4,4 | 12 % |
| Indométhacine 1 µM | 20,9 ± 4,9 | 37 % |

Le PMA ou phorbol-12-myristate-13-acetate, est un activateur de la protéine kinase C.

C'est un activateur non physiologique qui mime l'effet des cytokines dont l'IL-1βsur les KHNs, et incite la cellule à libérer une molécule lipidique, la prostaglandine 6ketoPGF_{1α}. Cette prostaglandine est dosée au moyen d'un test ELISA. Les deux activateurs IL-1β et PMA sont utilisés. L'indométhacine, analgésique de type aspirine est un anti-inflamatoire (AINS) qui stimule la réincorporation de l'acide arachidonique libre dans les triglycérides pour diminuer la libération des éicosanoïdes. Il est utilisé comme inhibiteur de la libération de 6-ketoPGF_{1α}.

Deux expériences ont été réalisées, la première consiste à prétraiter les KHN avec les oligorhamnosides pendant 4 heures, suivi de la stimulation pendant 2 heures, toujours en présence des oligorhamnosides. Les résultats sont résumés dans le tableau 6 précédent.

Une seconde expérience a été réalisée où le prétraitement avec les oligorhamnosides a été prolongé à 12 heures. Les résultats sont résumés dans le tableau 7 suivant.

**Tableau 7 : Résultats du traitement des KHN avec l'IL-1β et différentes concentrations en oligorhamnosides après 12 heures de prétraitement avec les oligorhamnosides**

| Traitement | Moy. 6-ketoPGF_{1α} | % inhibition |
|---|---|---|
| | (pg/mL/µg protéines) | |
| Témoin négatif | 19,5 ± 4,5 | |
| Témoin positif (IL-1b 1 ng/mL) | 35,5 ± 5,3 | |

| Stimulation 2 heures IL-1β 1 ng/mL | | |
|---|---|---|
| 2 mg/mL | 28,2 ±3,8 | 21 % |
| 1 mg/mL | 30,6 ±3,1 | 14 % |
| 0,5 mg/mL | 35,2 ±0,4 | / |
| 0,1 mg/mL | 31 ±5,1 | / |
| Indométhacine | 22,6 ± 3,4 | 36 % |

Dans ces conditions expérimentales, la libération du médiateur 6-ketoPGF1α par les KHN stimulés est faible. Il apparaît toutefois une légère inhibition de celle-ci lorsque les cellules sont traitées par les oligorhamnosides à forte concentration, supérieure à 1 mg/mL.

### ● Dosage de la PGE₂ libérée par les KHN stimulées par le PMA.

Les oligorhamnosides ont pu être évalués en tant qu'inhibiteur de la libération de PGE₂ dans les surnageants cellulaires. Ces produits sont mis en présence des KHN en même temps que le PMA à 1 ng/mL. Chaque condition testée est évaluée sur 4 puits de KHN pour la stimulation.

Après 24 heures de traitement, les résultats résumés dans le tableau 8 suivant représentent les moyennes des valeurs des concentrations en PGE₂ (pg/mL) données dans chacun des surnageants cellulaires stimulés ou non et rapportées à une quantité de cellules exprimées en µg.

**Tableau 8 : Pourcentage d'inhibition de la libération de PGE₂ en fonction de la concentration des dérivés rhamnosylés.**

| | 2 mg/mL | 1,5 mg/mL | 1 mg/mL | 0,5 mg/mL | 0,1 mg/mL |
|---|---|---|---|---|---|
| Oligorhamnosides | 58 % | | 25 % | | |
| | | 60 % | 53 % | 27 % | / |
| | 72% | | 49 % | | |

Les oligorhamnosides inhibent la libération de PGE₂ à des concentrations assez élevées : 2 mg/mL à 1 mg/mL avec une inhibition moyenne de 50 à 60 %. Pour des concentrations inférieures, son pouvoir inhibiteur diminue et devient nul à 0,1 mg/mL.

### ● Dosage des molécules LTB₄ et PGE₂ libérées par les polynucléaires neutrophiles humains

Les neutrophiles sont isolés de sang courant humain et purifiés par un procédé original.

Ils sont ensuite activés par un tampon de stimulation qui provoquera la libération des deux types de molécules lipidiques issues des voies cyclo-oxygénase et lipo-oxygénase, dérivées de l'acide arachidonique, à savoir le leucotriène LTB₄ et la prostaglandine PGE₂. Ce tampon de stimulation contient en particulier des ions Ca²⁺ et Mg²⁺. Ces dosages sont effectués au moyen d'un kit commercial qui répond à une méthode de dosage de type ELISA.

La viabilité cellulaire des neutrophiles traités avec les oligorhamnosides est évaluée au bleu trypan. Les viabilitées sont bonnes même aux concentrations les plus élevées en oligorhamnosides.

Les résultats représentent les moyennes des valeurs dosées dans deux surnageants de cellules stimulées au cours d'une même expérience.

Les résultats obtenus sur l'inhibition de la libération des molécules lipidiques sont résumés dans le tableau 9 suivant.

**Tableau 9 : Résultats du traitement des polynucléaires neutrophiles avec un tampon de stimulation et différentes concentrations en oligorhamnosides.**

| PGE₂ libérée (pg/mL) | | | |
|---|---|---|---|
| Traitement | Sans stimulation | Stimulation | % inhibition |
| Témoin négatif | 3,2±0,3 | 22,1±2,8 | |
| 1 mg/mL | 4±0,7 | 19,6±2,3 | 11 % |
| 0,5 mg/mL | 4,3±0,6 | 22,8 ± 0,9 | / |
| 0,1 mg/mL | 4,6±0,6 | 20±2,6 | / |
| Indométhacine 1 µM | 4,5±0 | 6,8±1 | 69 % |

| LTB₄ libérée (pg/mL) | | | |
|---|---|---|---|
| Traitement | Sans stimulation | Stimulation | % inhibition |
| Témoin négatif | 24,3 | 2494 ±105 | |
| 1 mg/mL | 19,8 | 1857±97 | 26 % |
| 0,5 mg/mL | 33,3 | 2199 ±122 | 12 % |
| 0,1 mg/mL | 34,1 | 2073 ± 169 | 17 % |
| NDGA 0,1 µM | / | 684±185 | 73 % |

L'indométhacine est utilisé comme inhibiteur spécifique de la libération de PGE₂ et l'acide Nordihydroguaiarétique comme inhibiteur spécifique de la libération de LTB₄.

Ce tableau permet de conclure qu'il y a une très faible inhibition de la libération de PGE₂ (11 %) observée après le traitement des neutrophiles avec les oligorhamnosides. Cependant, en présence d'une concentration de 1 mg/mL d'oligorhamnosides, une inhibition de la libération de LTB₄ de 26 % est obtenue et confirmée.

En conclusion, les oligorhamnosides à une concentration de 1 mg/mL assurent une inhibition de la libération de l'IL-8 de 27 à 28 % par des KHN dans deux expériences indépendantes cependant la libération de 6-ketoPGF1α par les KHN est très légèrement diminuée. A plus fortes concentrations, de 1 à 2 mg/mL les oligorhamnosides inhibe en moyenne de 50 à 60 % la libération de PGE₂ par des KHN. Ces mêmes molécules à une concentration de 1 mg/mL contribuent à une inhibition de 21 et 26 % de la libération de LTB₄ par des neutrophiles dans deux expériences indépendantes. Ces molécules n'ont aucun effet sur la libération de PGE₂ par des neutrophiles humains.

## Revendications

1. Procédé de préparation d'oligorhamnosides, **caractérisé en ce qu'**il comprend les étapes successives suivantes :
a) autocondensation du rhamnose en une seule étape réactionnelle dans l'acétonitrile en présence d'un catalyseur acide et précipitation des oligorhamnosides ainsi formés ; puis
b) récupération du précipité obtenu suite à l'étape a), comprenant les oligorhamnosides, par filtration.

2. Procédé selon la revendication 1, **caractérisé en ce que** la température du mélange réactionnel, lors de l'étape a), est comprise entre 20 et 120°C, avantageusement entre 35 et 75°C.

3. Procédé selon l'une quelconque des revendications précédentes, **caractérisé en ce que** le catalyseur acide est choisi dans le groupe constitué par l'acide chlorhydrique, l'acide sulfurique, l'acide phosphorique, l'acide ortho-, méta-, et para-toluène sulfonique, l'acide benzène-sulfonique, les acides benzène-sulfoniques substitués, l'acide méthane-sulfonique, les acides de Lewis, notamment le chlorure de zinc ou le chlorure ferrique, des argiles acides, notamment la montmorillonite K-10, des résines acides synthétiques, les zéolithes ou leurs combinaisons.

4. Procédé selon l'une quelconque des revendications précédentes, **caractérisé en ce que** l'eau formée pendant la réaction d'autocondensation de l'étape a) est éliminée physiquement ou chimiquement.

5. Procédé selon la revendication 4, **caractérisé en ce que** la technique d'élimination de l'eau comprend l'utilisation d'un agent de dessiccation choisi dans le groupe constitué par les carbonates, les sulfates, le chlorure de calcium, le pentaoxyde de phosphore, des tamis moléculaires ou des combinaisons de ces divers agents de dessiccation.

6. Procédé selon l'une quelconque des revendications précédentes, **caractérisé en ce que** l'étape a) est réalisée à pression atmosphérique et sous atmosphère d'un gaz inerte, tel que l'argon ou l'azote.

7. Procédé selon l'une quelconque des revendications 1 à 6, **caractérisé en ce que** l'étape a) est réalisée à pression réduite, en autoclave.

8. Procédé selon l'une quelconque des revendications précédentes, **caractérisé en ce que** préalablement à l'étape b), le mélange réactionnel est refroidi jusqu'à une température comprise entre la température de réaction de condensation et 0°C.

9. Procédé selon la revendication 8, **caractérisé en ce que** le mélange réactionnel est refroidi jusqu'à la température ambiante, avantageusement jusqu'à 20°C.

10. Procédé selon l'une quelconque des revendications précédentes, **caractérisé en ce que** le précipité récupéré suite à l'étape b) est lavé à l'acétronitrile.

11. Procédé selon l'une quelconque des revendications précédentes, **caractérisé en ce que** l'acétonitrile compris dans le filtrat obtenu suite à l'étape b) est évaporé afin de récupérer un second précipité comprenant des oligorhamnosides.

12. Composition comprenant un mélange d'oligorhamnosides susceptible d'être obtenu par le procédé selon l'une quelconque des revendications 1 à 10, **caractérisée en ce que** lesdits oligorhamnosides contiennent de 2 à 12 motifs rhamnoses, avantageusement de 2 à 9 motifs rhamnoses.

13. Composition selon la revendication 12, **caractérisée en ce que** la distribution des oligorhamnosides en fonction de leur degré de polymérisation suit approximativement une loi de distribution type loi de Poisson.

14. Composition selon les revendications 12 ou 13, **caractérisée en ce que** les motifs rhamnoses ont jusqu'à trois de leurs fonctions hydroxyles qui sont impliquées dans la formation de liaisons glycosidiques.

15. Médicament comprenant une composition telle que définie à l'une quelconque des revendications 12 à 14.

16. Médicament selon la revendication 15, destiné à réguler les mécanismes inflammatoires.

17. Médicament selon la revendication 15 ou 16, destiné à la prévention ou au traitement des réactions ou pathologies allergiques, inflammatoires, immunitaires de la peau et/ou des muqueuses.

18. Médicament selon l'une quelconque des revendications 15 à 17, destiné à inhiber la réponse immune liée au stress inflammatoire.

19. Médicament selon l'une quelconque des revendications 15 à 18, destiné à inhiber l'activation des leucocytes, la sécrétion des cytokines kératinocytaires, le phénomène d'hyperplasie kératinocytaire, le processus d'apprêtement de l'antigène par les cellules dendritiques de la peau, la maturation des cellules présentatrices d'antigène, le phénomène de reconnaissance entre les lymphocytes et les cellules présentatrices d'antigène.

20. Médicament selon l'une quelconque des revendications 15 à 19, destiné à la prévention ou au traitement des maladies choisies dans le groupe constitué de l'eczéma atopique et/ou de contact, des dermatoses inflammatoires, des dermatites irritatives, de l'acné, des maladies auto-immunes telles que le psoriasis, de la photo-immuno-suppression, du vitiligo, du pityriasis, des sclérodermies, de l'arthrite rhumatoïde, de la maladie de Crohn ou du rejet de greffe.

21. Médicament selon l'une quelconque des revendications 15 à 20, destiné à la prévention et au traitement des dérives inflammatoires chroniques liées au vieillissement et ses conséquences.

22. Médicament selon la revendication 21, destiné à la prévention ou au traitement des maladies choisies dans le groupe constitué par les sensibilisations anaphylactiques, les anomalies pigmentaires de la peau, l'hypervascularisation dermique, les fissurations inflammatoires.

23. Médicament selon l'une quelconque des revendications 15 à 22, destiné à diminuer le caractère allergisant et/ou irritant d'une composition ou d'un parfum.

24. Médicament selon l'une quelconque des revendications 15 à 23, **caractérisé en ce qu'**il comprend 0,001 à 50 % en poids d'oligorhamnosides.

25. Composition selon l'une quelconque des revendications 12 à 14 pour le traitement cosmétique des peaux et/ou des muqueuses sensibles, irritées, intolérantes, à tendance allergique, âgées, soumises à un signal danger, présentant un trouble de la barrière cutanée, présentant des rougeurs cutanées ou présentant un déséquilibre immunologique non pathologique lié au vieillissement intrasèque, extrinsèque ou hormonal.

26. Composition selon l'une quelconques des revendications 12 à 14 pour retarder le vieillissement naturel de la peau et/ou pour prévenir le vieillissement accéléré de la peau soumise aux agressions extérieures, notamment pour prévenir le vieillissement photo-induit de la peau.

## Claims

1. A method for the preparation of oligorhamnosides, wherein it comprises the following successive steps:
a) self-condensation of rhamnose in a single reaction step in acetonitrile in the presence of an acid catalyst and precipitation of the oligorhamnosides thus formed; then
b) recovery by filtration of the precipitate obtained following step a) comprising the oligorhamnosides.

2. The method according to claim 1, wherein the temperature of the reaction mixture, during step a), lies between 20 °C and 120 °C, advantageously between 35 °C and 75 °C.

3. The method according to either of the preceding claims, wherein the acid catalyst is chosen from the group comprised of hydrochloric acid, sulfuric acid, phosphoric acid, ortho-, meta- and para-toluenesulfonic acid, benzene-sulphonic acid, substituted benzene-sulphonic acids, methanesulphonic acid, Lewis acids, in particular zinc chloride and ferric chloride, clay acids, in particular montmorillonite K-10, synthetic resin acids, zeolites and combinations thereof.

4. The method according to any of the preceding claims, wherein the water formed during the self-condensation reaction of step a) is eliminated physically or chemically.

5. The method according to claim 4, wherein the water elimination technique comprises the use of a desiccation agent chosen among the group consisting of the carbonates, the sulfates, calcium chloride, phosphorus pentoxide, the molecular sieves or combinations of these various desiccation agents.

6. The method according to any of the preceding claims, wherein step a) is carried out at atmospheric pressure and under atmosphere of an inert gas, such as argon or nitrogen.

7. The method according to any of the claims 1 to 6, wherein step a) is carried out at reduced pressure, in an autoclave.

8. The method according to any of the preceding claims, wherein prior to step b), the reaction mixture is cooled to a temperature in the range between the condensation reaction temperature and 0 °C.

9. The method according to claim 8, wherein the reaction mixture is cooled to ambient temperature, advantageously to 20 °C.

10. The method according to any of the preceding claims, wherein the precipitate recovered following step b) is washed with acetonitrile.

11. The method according to any of the preceding claims, wherein the acetonitrile included in the filtrate obtained following step b) is evaporated in order to recover a second precipitate containing oligorhamnosides.

12. A composition comprising a mixture of oligorhamnosides likely to be obtained by a method according to any of the claims 1 to 10, wherein the aforementioned oligorhamnosides contain from 2 to 12 rhamnose motifs, advantageously from 2 to 9 rhamnose motifs.

13. The composition according to claim 12, wherein the distribution of oligorhamnosides as a function of their degree of polymerization roughly follows a Poisson distribution.

14. The composition according to claims 12 or 13, wherein the rhamnose motifs have up to three of their hydroxyl functions implicated in the formation of glycosidic bonds.

15. A medicament containing a composition as defined in any of the claims 12 to 14.

16. The medicament according to claim 15, intended to regulate inflammatory mechanisms.

17. The medicament according to claim 15 or 16, intended for the prevention or treatment of allergic, inflammatory or immune reactions or pathologies of the skin and/or mucous membranes.

18. The medicament according to any of the claims 15 to 17, intended to inhibit the immune response related to inflammatory stress.

19. The medicament according to any of the claims 15 to 18, intended to inhibit leukocyte activation, secretion of keratinocytic cytokines, keratinocytic hyperplasia phenomenon, antigen processing by the dendritic cells of the skin, maturation of antigen-presenting cells, and recognition phenomenon between lymphocytes and antigen-presenting cells.

20. The medicament according to any of the claims 15 to 19, intended for the prevention or treatment of diseases chosen from the group comprised of atopic and/or contact eczema, inflammatory dermatoses, irritant dermatitis, acne, autoimmune diseases such as psoriasis, photo-immunosuppression, vitiligo, pityriasis, sclerodermas, rheumatoid arthritis, Crohn's disease and graft rejection.

21. The medicament according to any of the claims 15 to 20, intended for the prevention and treatment of age-related chronic inflammatory problems and their consequences.

22. The medicament according to claim 21, intended for the prevention or treatment of diseases chosen from the group comprised of anaphylactic sensitivities, pigmentary anomalies of the skin, dermal hypervascularity and inflammatory fissuring.

23. The medicament according to any of the claims 15 to 22, intended to reduce the allergenic and/or irritant character of a composition or perfume.

24. The medicament according to any of the claims 15 to 23, wherein it contains from 0.001% to 50% by weight of oligorhamnosides.

25. The composition according to any of the claims 12 to 14 for the cosmetic treatment of skin and/or mucous membranes that are sensitive, irritated, intolerant, of an allergic tendency, aged, exhibiting danger signs, exhibiting a disorder of the cutaneous barrier, exhibiting cutaneous redness or exhibiting a non-pathological immunological imbalance related to intrinsic, extrinsic or hormonal aging.

26. The composition according to any of the claims 12 to 14 to slow the natural aging of the skin and/or to prevent the accelerated aging of skin subjected to external attacks, in particular to prevent photo-induced aging of the skin.

## Patentansprüche

1. Verfahren zur Herstellung von Oligorhamnosiden, **dadurch gekennzeichnet, dass** es die folgenden aufeinander folgenden Schritte umfasst:
a) Autokondensation der Rhamnose in einem einzigen Reaktionsschritt in Acetonitril in Gegenwart eines sauren Katalysators und Ausfällen der so gebildeten Oligorhamnoside; dann
b) Gewinnung des nach dem Schritt a) erhaltenen Niederschlags, welcher die Oligorhamnoside umfasst, durch Filtration.

2. Verfahren nach Anspruch 1, **dadurch gekennzeichnet, dass** die Temperatur der Reaktionsmischung während des Schritts a) zwischen 20 und 120°C, vorteilhafterweise zwischen 35 und 75°C liegt.

3. Verfahren nach einem der vorangegangenen Ansprüche, **dadurch gekennzeichnet, dass** der saure Katalysator ausgewählt wird in der Gruppe, welche aus Salzsäure, Schwefelsäure, Phosphorsäure, ortho-, meta- und para-Toluolsulfonsäure , Benzolsulfonsäure, den substituierten Benzolsulfonsäuren, Methansulfonsäure, den Lewis-Säuren, insbesondere Zinkchlorid oder Eisen(III)-chlorid, sauren Tonen, insbesondere dem Montmorillonit K-10, sauren Kunstharzen, den Zeolithen oder deren Kombinationen gebildet wird.

4. Verfahren nach einem der vorangegangenen Ansprüche, **dadurch gekennzeichnet, dass** das während der Autokondensationsreaktion des Schritts a) gebildete Wasser physisch oder chemisch entfernt wird.

5. Verfahren nach Anspruch 4, **dadurch gekennzeichnet, dass** die Technik zur Entfernung des Wassers die Verwendung eines Trocknungsmittels, welches in der Gruppe, die aus den Carbonaten, den Sulfaten, dem Chlorid von Calcium, Phosphorpentoxid, Molekularsieben oder Kombinationen von diesen unterschiedlichen Trocknungsmitteln gebildet wird, ausgewählt wird, umfasst.

6. Verfahren nach einem der vorangegangenen Ansprüche, **dadurch gekennzeichnet, dass** der Schritt a) bei Atmosphärendruck und unter der Atmosphäre eines Inertgases, wie Argon oder Stickstoff, ausgeführt wird.

7. Verfahren nach einem der Ansprüche 1 bis 6, **dadurch gekennzeichnet, dass** der Schritt a) bei verringertem Druck in einem Autoklaven ausgeführt wird.

8. Verfahren nach einem der vorangegangenen Ansprüche, **dadurch gekennzeichnet, dass** vor dem Schritt b) die Reaktionsmischung bis auf eine Temperatur zwischen der Kondensationsreaktionstemperatur und 0°C eingeschlossen abgekühlt wird.

9. Verfahren nach Anspruch 8, **dadurch gekennzeichnet, dass** die Reaktionsmischung bis auf Umgebungstemperatur, vorteilhafterweise bis auf 20°C abgekühlt wird.

10. Verfahren nach einem der vorangegangenen Ansprüche, **dadurch gekennzeichnet, dass** der nach dem Schritt b) gewonnene Niederschlag mit Acetonitril gewaschen wird.

11. Verfahren nach einem der vorangegangenen Ansprüche, **dadurch gekennzeichnet, dass** das Acetonitril, das in dem nach dem Schritt b) erhaltenen Filtrat enthalten ist, verdampft wird, um einen zweiten Niederschlag, welcher Oligorhamnoside umfasst, zu gewinnen.

12. Zusammensetzung, umfassend eine Mischung von Oligorhamnosiden, welche durch das Verfahren nach einem der Ansprüche 1 bis 10 erhalten werden kann, **dadurch gekennzeichnet, dass** die Oligorhamnoside 2 bis 12 Rhamnose-Motive, vorteilhafterweise 2 bis 9 Rhamnose-Motive enthalten.

13. Zusammensetzung nach Anspruch 12, **dadurch gekennzeichnet, dass** die Verteilung der Oligorhamnoside abhängig von ihrem Polymerisationsgrad näherungsweise einem Verteilungsgesetz vom Typ der Poisson-Verteilung folgt.

14. Zusammensetzung nach den Ansprüchen 12 oder 13, **dadurch gekennzeichnet, dass** bei den Rhamnose-Motiven bis zu drei von deren Hydroxyl-Funktionen an der Bildung von glycosidischen Bindungen beteiligt sind.

15. Arzneimittel, welches eine Zusammensetzung, wie in einem der Ansprüche 12 bis 14 definiert, umfasst.

16. Arzneimittel nach Anspruch 15, welches dazu bestimmt ist, die Entzündungsmechanismen zu regulieren.

17. Arzneimittel nach Anspruch 15 oder 16, welches für die Verhütung oder für die Behandlung von allergischen Reaktionen oder Pathologien, entzündlichen Reaktionen oder Pathologlen, Immunreaktionen oder -erkrankungen der Haut und/oder der Schleimhäute bestimmt ist.

18. Arzneimittel nach einem der Ansprüche 15 bis 17, welches dazu bestimmt ist, die mit dem Entzündungsstress verbundene Immunantwort zu hemmen.

19. Arzneimittel nach einem der Ansprüche 15 bis 18, welches dazu bestimmt ist, die Aktivierung der Leukozyten, die Sekretion der keratinozytären Zytokine, das Phänomen von keratinozytärer Hyperplasie, den Prozess der Antigenprozessierung durch die dendritischen Zellen der Haut, die Reifung der Antigen-präsentierenden Zellen, das Erkennungsphänomen zwischen den Lymphozyten und den Antigen-präsentierenden Zellen zu hemmen.

20. Arzneimittel nach einem der Ansprüche 15 bis 19, welches für die Verhütung oder für die Behandlung der Erkrankungen, die in der Gruppe bestehend aus dem atopischen Ekzem und/oder Kontaktekzern, entzündlichen Dermatosen, nicht-allergischen Kontaktdermatitiden, Akne, Autoimmunerkrankungen, wie Psoriasis, der Photoimmunsuppression, Vitiligo, Pityriasis, Sklerodermien, rheumatoider Arthritis, Morbus Crohn oder der Transplantatabstoßung ausgewählt werden, bestimmt ist.

21. Arzneimittel nach einem der Ansprüche 15 bis 20, welches für die Verhütung und die Behandlung der mit der Alterung und ihren Folgen verbundenen chronischen entzündlichen Folgeerscheinungen bestimmt ist.

22. Arzneimittel nach Anspruch 21, das für die Verhütung oder die Behandlung der Erkrankungen, die in der Gruppe bestehend aus den anaphylaktischen Sensibilisierungen, den Pigmentanomalien der Haut, der Hypervaskularisation der Haut, den entzündlichen Riss- oder Schrundenbildungen ausgewählt wird, bestimmt ist.

23. Arzneimittel nach einem der Ansprüche 15 bis 22, welches dazu bestimmt ist, den allergisierenden und/oder reizenden Charakter einer Zusammensetzung oder eines Parfums zu verringern.

24. Arzneimittel nach einem der Ansprüche 15 bis 23, **dadurch gekennzeichnet, dass** es 0,001 bis 50 Gew.-% Oligorhamnoside umfasst.

25. Zusammensetzung nach einem der Ansprüche 12 bis 14 für die kosmetische Behandlung von Haut und/oder Schleimhäuten, die empfindlich sind, gereizt sind, überempfindlich sind, eine Allergieneigung aufweisen, gealtert sind, einem Gefahrensignal ausgesetzt sind, eine Störung der Hautbarriere aufweisen, Hautrötungen aufweisen oder ein nicht-pathologisches immunologisches Ungleichgewicht, welches mit der endogenen, exogenen oder hormonellen Alterung verbunden ist, aufweisen.

26. Zusammensetzung nach einem der Ansprüche 12 bis 14, um die natürliche Alterung der Haut zu verzögern und/oder um die beschleunigte Alterung der Haut, die Angriffen von Außen ausgesetzt ist, zu verhüten, insbesondere um die Licht-induzierte Alterung der Haut zu verhüten.
